(19) Europäisches Patentamt
European Patent Office
Office européen des brevets

(11) EP 1 073 686 B1

(12) FASCICULE DE BREVET EUROPEEN

(45) Date de publication et mention de la délivrance du brevet:
**14.05.2008 Bulletin 2008/20**

(21) Numéro de dépôt: **99915807.4**

(22) Date de dépôt: **21.04.1999**

(51) Int Cl.:
***C08G 18/79*** *(2006.01)* ***C08G 18/78*** *(2006.01)*
***C09D 175/04*** *(2006.01)*

(86) Numéro de dépôt international:
**PCT/FR1999/000950**

(87) Numéro de publication internationale:
**WO 1999/055756 (04.11.1999 Gazette 1999/44)**

# (54) PROCEDE DE PREPARATION D'ISOCYANATES POLYFONCTIONNELS TRICONDENSATS DE FAIBLE VISCOSITE

VERFAHREN ZUR HERSTELLUNG VON TRIMEREN POLYISOCYANATEN MIT GERINGER VISKOSITÄT

METHOD FOR PREPARING LOW VISCOSITY TRICONDENSATE POLYFUNCTIONAL ISOCYANATES

(84) Etats contractants désignés:
**AT BE CH CY DE DK ES FI FR GB GR IE IT LI LU MC NL PT SE**

(30) Priorité: **24.04.1998 FR 9805170**

(43) Date de publication de la demande:
**07.02.2001 Bulletin 2001/06**

(73) Titulaire: **RHODIA CHIMIE**
**92408 Courbevoie Cédex (FR)**

(72) Inventeurs:
- **CHARRIERE, Eugénie**
  **F-69000 Lyon (FR)**
- **BERNARD, Jean-Marie**
  **F-69440 Mornant (FR)**
- **REVELANT, Denis**
  **F-69740 Genas (FR)**

(74) Mandataire: **Bernasconi, Jean Raymond et al**
**c/o Cabinet Lavoix,**
**2, Place d'Estienne d'Orves**
**75441 Paris Cedex 09 (FR)**

(56) Documents cités:
**EP-A- 0 277 353** **EP-A- 0 649 866**
**US-A- 5 258 482**

Remarques:
Le dossier contient des informations techniques présentées postérieurement au dépôt de la demande et ne figurant pas dans le présent fascicule.

EP 1 073 686 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

L'invention concerne la préparation de compositions polyisocyanates de viscosité abaissée qui sont destinées notamment à des compositions de revêtement, en particulier de peinture, à deux composants.

Les polyisocyanates sont utilisés largement dans l'industrie du revêtement, en particulier des peintures, en raison de leurs nombreuses propriétés. Il est notamment connu de mettre en oeuvre, en tant que durcisseurs, des polyisocyanates comprenant des groupes isocyanurates en raison de leur capacité de réticulation.

Toutefois, les compositions de ce type obtenues par trimérisation d'un isocyanate ont une viscosité relativement élevée qui nécessite l'utilisation d'une quantité substantielle de solvant.

Or, les réglementations nouvelles en matière de contrôle de l'environnement imposent la diminution des composés organiques volatils.

Pour répondre à ces exigences, une des possibilités consiste à limiter le taux de transformation des isocyanates de départ, notamment des diisocyanates afin de minimiser la formation de composés lourds (polycondensats de plus haut degré de polymérisation, plus particulièrement comprenant plus d'un motif trimère), présents dans le milieu de trimérisation responsables de l'augmentation de la viscosité. A cet effet, on réduit la quantité de catalyseur pour un temps de réaction fixé, ou on diminue la durée de réaction pour une quantité de catalyseur donnée, afin d'augmenter le rapport cyclotrimères vrais/composés lourds.

La Demanderesse commercialise déjà des produits de ce type, HDT (Hexaméthylène Diisocyanate Trimère) et HDB (Hexaméthylène Diisocyanate Biuret), désignés sous l'acronyme anglo-saxon LV par "Low Viscosity"

Les inconvénients de ces modes opératoires sont, dans le premier cas, une baisse importante de la productivité et dans le second. une augmentation du coût due à la quantité de catalyseur utilisée pour une quantité donnée d'isocyanurates.

Il a également été proposé dans les demandes de brevets européens EP 524 500 et EP 524 501 de réaliser une réaction d'allophanatation sur un mélange de trimérisation ou de réaliser la trimérisation en présence d'alcools, ce qui conduit à des mélanges polyisocyanates à fonctions isocyanurates revendiqués comme présentant une basse viscosité.

Par ailleurs, on n'a pas décrit le fait que les composés biurets peuvent voir leur viscosité diminuée par addition d'allophanates, ni même par allophanatation concomitante ou consécutive.

Les allophanates sont obtenus dans le milieu réactionnel par réaction d'un composé à fonction alcool sur un isocyanate, puis par réaction de la fonction carbamate ainsi obtenue sur une nouvelle molécule d'isocyanate simultanément voire consécutivement à la réaction de trimérisation.

Les procédés d'obtention de compositions polyisocyanates à motifs isocyanurates ayant une teneur significative en fonctions allophanates ont jusqu'à présent consisté à soumettre le mélange de trimérisation obtenu après cyclotrimérisation catalytique partielle des isocyanates de départ à une réaction d'allophanatation consécutive en présence d'un alcool notamment de butanol, puis à éliminer ultérieurement les isocyanates de départ par distillation sous vide.

Les compositions obtenues selon les procédés décrits dans les demandes de brevet ci-dessus mentionnées ont généralement une viscosité quelque peu abaissée par rapport à celle des compositions ne comprenant pas d'allophanates.

Au cours de l'étude qui a mené à la présente invention, il a été montré, de manière surprenante, qu'il était possible d'obtenir pour des compositions présentant une même teneur en (poly)isocyanates trifonctionnels, trimères (comprenant au moins un groupe isocyanurate et/ou biuret) une viscosité abaissée. Ainsi, par rapport aux compositions de (poly)isocyanate(s) isocyanurate(s) comprenant des allophanates de l'art antérieur, en réalisant de manière séparée la réaction de (cyclo)trimérisation catalytique et la réaction d'allophanatation, on obtient une viscosité significativement abaissée.

Il a été démontré que la présence d'allophanates d'isocyanates polyfonctionnels (ayant une fonctionnalité d'au moins trois), notamment d'allophanates contenant des groupes isocyanurates dans la composition finale obtenue selon les méthodes de l'art antérieur augmentait de façon significative et néfaste la viscosité de cette composition et qu'au contraire, en procédant de sorte qu'il ne se forme pas d'allophanates d'isocyanates polyfonctionnels, la viscosité de la composition finale était significativement diminuée par rapport à la viscosité de la composition d'isocyanates polyfonctionnels obtenue à l'issue de la trimérisation des isocyanates de départ.

On utilisera dans la suite du texte l'expression "comportant des groupes isocyanurates" comme un paradigme des composés polyfonctionnels.

L'invention a ainsi pour but de fournir des compositions isocyanates polyfonctionnels comportant des tricondensats de préférence à fonction biuret et/ou isocyanurate, ladite composition comprenant des composés à fonctions allophanates et présentant une viscosité significativement abaissée, de préférence d'au moins environ ¼, avantageusement environ 1/3, de manière plus avantageuse encore d'environ 2/5, en l'absence de solvant, par rapport à la même composition ne comprenant pas de composés à fonctions allophanates, pour une température donnée.

Dans la présente description, le terme "environ" signifie que la valeur donnée correspond à un arrondi mathématique et que les éventuels zéros les plus à droite sont des zéros de position et non pas des chiffres significatifs.

A cet effet, l'invention a pour objet un procédé de préparation d'une composition d'isocyanates polyfonctionnels tricondensats, de préférence comprenant au moins un groupe isocyanurate et/ou biuret, consistant à ajouter à un isocyanate polyfonctionnel tricondensat, ou à un mélange de différents isocyanates polyfonctionnels tricondenstas, obtenus par (cyclo)condensation, notamment (cyclo)trimérisation d'un ou plusieurs isocyanates monomères identiques ou différents et éventuellement d'un autre monomère, un allophanate d'un ou plusieurs isocyanates identiques ou différents, ou un mélange de différents allophanates, les isocyanates ou mélanges d'isocyanates monomères utilisés pour la préparation du(des) isocyanate(s) polyfonctionnel(s) étant identiques ou différents du(des) isocyanate(s) ou du mélange d'isocyanates utilisés pour la préparation du(des) allophanate(s) et le(s) allophanate(s) est(sont) préparé(s) à partir d'alcools alkyliques à chaîne linéaire en $C_4$-$C_8$.

Les isocyanates polyfonctionnels (cyclo)tricondensats de l'invention répondent à la formule générale suivante :

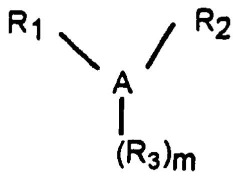

(I)

dans laquelle A représente

- un groupe isocyanurate de formule

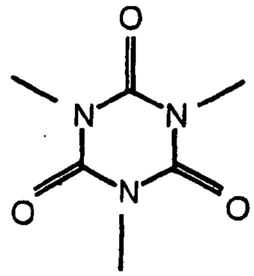

;

- un de ses dérivés à squelette imino-oxadiazine-dione de formule générale suivante :

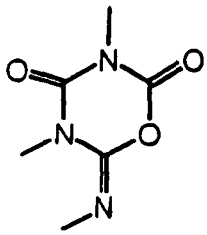

- un de ses dérivés à squelette oxadiazine-trione de formule générale suivante

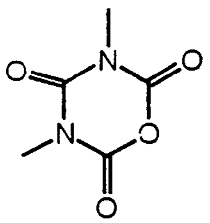

- un groupe biuret de formule

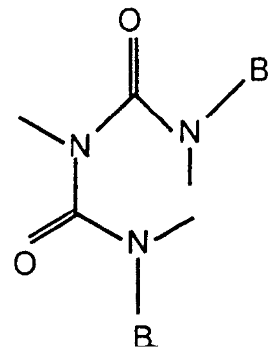

B étant H ou un groupe hydrocarboné, c'est-à-dire contenant du carbone et de l'hydrogène ainsi qu'éventuellement d'autres atomes (O, S, Si, ..) ayant de préférence 1 à 20 atomes de carbone ; ou

- un groupe de formule :

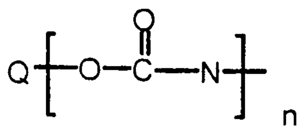

et dans laquelle $R_1$, $R_2$, $R_3$ identiques ou différents représentent un groupe hydrocarboné, notamment aliphatique, cycloaliphatique, hétérocyclique ou aromatique, comprenant une fonction isocyanate vraie ou dérivée.
Q est un groupe hydrocarboné, de préférence alcoyle, tel que défini pour $R_1$ à $R_3$,
m est un nombre entier de 0 à 2. et
n est un nombre entier de 3 à 4.

On entend par fonction isocyanate dérivée les fonctions carbamate, urée, biuret, uréthane, urétinedione, isocyanurate et isocyanate masquée.

Les isocyanates polyfonctionnels tricondensats peuvent être des homotricondensats (lorsque $R_1$, $R_2$ et $R_3$ sont identiques) ou des hétérotricondensats lorsque l'un au moins de $R_1$, $R_2$ et $R_3$ est différent des autres).

Les mélanges d'isocyanates polyfonctionnels tricondensats sont définis comme étant une combinaison d'isocyanates polyfonctionnels homotricondensats différents, d'isocyanates polyfonctionnels hétérotricondensats différents ou un mélange des deux catégories.

On parlera d'isocyanate polyfonctionnel tricondensat vrai, lorsque $R_1$, $R_2$, $R_3$ identiques ou différents représentent un groupe -A-X, A étant une chaîne hydrocarbonée telle que définie ci-dessus, c'est-à-dire comportant au moins du carbone et de l'hydrogène et X un atome d'hydrogène ou un groupe NCO.

On préfère que X représente un groupe NCO.

En d'autres termes, par isocyanate polyfonctionnel tricondensat vrai, on entend les produits de (cyclo)condensation théorique obtenus par condensation de trois moles de monomères, avantageusement d'isocyanates, de préférence diisocyanates voire triisocyanates (identiques ou différents), à l'exception des composés provenant de la condensation de plus de quatre monomères et/ou comportant des groupes allophanates, ainsi que les oligomères isocyanurates

obtenus par oligomérisation de (poly)isocyanates isocyanurates.

Les allophanates de la présente invention répondent à la formule générale II :

$$R_4 - NC(O)O - R_5 \quad (II)$$
$$\qquad | $$
$$\qquad CO - NHR_6$$

dans laquelle :

- $R_4$ et $R_6$ identiques ou différents représentent un groupe hydrocarboné, notamment aliphatique, cycloaliphatique, hétérocyclique ou aromatique, tel que défini précédemment comprenant une fonction isocyanate vraie ou dérivée.
- $R_5$ représentant un groupe alcoyle, à savoir le reste d'un composé alcool après enlèvement de la fonction OH, à chaîne linéaire en $C_4$-$C_8$.

On entend par fonction isocyanate dérivée, dans ce cas, les fonctions carbamate, urée, biuret, uréthanne, uretinedione, isocyanate masquée, allophanate, à l'exclusion de la fonction isocyanurate.

Lorsque $R_4$ est identique à $R_6$, on parlera d'homo-allophanates, obtenus par condensation sur un carbamate formé par réaction d'un isocyanate de formule $R_4NCO$ avec un alcool de formule $R_5OH$, d'un second isocyanate, de formule $R_6NCO$, $R_6$ étant identique à $R_4$.

Les allophanates peuvent également être obtenus par condensation sur le carbamate d'un second isocyanate $R_6NCO$, $R_6$ étant différent de $R_4$, auquel cas on parlera d'hétéro-allophanates.

Avantageusement, on ajoute un mélange d'allophanates comprenant au moins 1/4, avantageusement au moins 1/3, de préférence au moins 2/5 (en masse) d'allophanate(s) primaire(s), d'un monoalcool, à chaîne linéaire en $C_4$-$C_8$.

Le mélange peut également comprendre des bis-allophanates, tris-allophanates et allophanates lourds, ainsi que de manière minoritaire, du carbamate de(s) isocyanate(s) ($R_4NCO$ et/ou $R_6NCO$) et d'alcool ($R_5OH$).

Il est très souhaitable que le mélange comprenne au plus 1/2 (en masse), avantageusement au plus 1/3, de préférence 1/6, d'allophanates lourds (comprenant plus de deux fonctions allophanates).
Il est à souligner que les bis-allophanates et les tris-allophanates en particulier les bis-allophanates et tris-allophanates de monoalcools, lorsqu'ils sont ajoutés à des polyisocyanates polyfonctionnels tricondensats à titre d'agents déviscosants ne constituent généralement pas une cause d'augmentation significative de la viscosité, à la différence des allophanates lourds.

Les bis-allophanates et tris-allophanates concourent même aux propriétés déviscosantes du mono-allophanate.

Dans le cadre de l'invention, on peut ajouter à la composition d'isocyanates polyfonctionnels tricondensats une composition d'allophanates comprenant uniquement des bis-allophanates et tris-allophanates.

Ce mode de réalisation n'est toutefois pas préféré en raison de la difficulté de préparer une composition exempte de mono-allophanate.

Ainsi, dans le cas d'un alcool monofonctionnel en particulier, la somme des mono-, bis- et tris-allophanates ajoutés comme agents déviscosants à la composition d'isocyanates polyfonctionnels tricondensats est avantageusement d'au moins 2/3 et de préférence d'au moins 75 %, de préférence d'au moins 90 %, en poids par rapport au poids total de la composition d'allophanates après élimination des monomères n'ayant pas réagi.

La quantité de bis-allophanates peut aller jusqu'à 10 % voire 20 % en poids du total de la composition d'allophanates sans modifier de manière substantielle les propriétés déviscosantes de celle-ci.

Parallèlement, la quantité de tris-allophanates peut représenter jusqu'à 30 % en poids du total de la composition d'allophanates. Toutefois, on préfère que celle-ci n'excède pas 20 %, de préférence 15 % en poids.

La composition d'allophanates ajoutée à la composition d'isocyanates polyfonctionnels tricondensats possède encore une très bonne propriété déviscosante lorsque le rapport

$$\frac{\text{fonctions bis-allophanates + fonctions tris-allophanates}}{\text{fonctions mono-allophanates}} \text{ est égal ou supérieur à 0,1,}$$

et peut aller jusqu'à 0,3 voire 0,5.

Compte tenu de ce que la composition (poly)isocyanate à fluidifier contient peu d'allophanates, les caractéristiques ci-dessus se retrouvent dans les rapports entre allophanates du mélange final.

Suivant la viscosité de la composition à fluidifier, il convient d'utiliser une composition allophanate dont la viscosité à 25°C est au plus égale à 45% avantageusement 35%, de préférence 30%, de la viscosité de la composition (poly)isocyanate à fluidifier.

Par allophanate "primaire", on entend le produit de réaction moléculaire théorique attendu de la réaction entre le(s) isocyanate(s) et l'alcool correspondant au produit de réaction de deux moles de fonction isocyanate par mole de fonction alcool.

L'allophanate primaire est obtenu à partir d'une seule molécule d'alcool porteuse d'une fonction hydroxyle transformée en fonction allophanate.

Ainsi, l'allophanate est vrai lorsque la condition suivante est vérifiée :

$$\frac{\text{Nombre total de fonctions allophanates par molécule de composé porteuses de fonctions allophanate(s)}}{\text{Nombre de chaînes isocyanates identiques ou différentes engagées dans les fonctions allophanates portées par la molécule de composé porteuses de fonctions allophanate(s).}} = 1/2$$

Par mono-allophanate, on entend le produit de réaction théorique, d'une mole d'isocyanate $R_4NCO$ sur une mole d'alcool ($R_5OH$) et d'une mole d'isocyanate $R_6NCO$ sur la fonction carbamate ainsi formée.

Un bis-allophanate est une molécule qui se caractérise par le fait qu'elle comporte deux fonctions allophanates, séparées par une chaîne au moins partiellement hydrocarbonée.

Pour ces molécules, le rapport indiqué ci-dessus est supérieur à ½, en ne prenant pas en compte pour le calcul de ce rapport les molécules allophanates possédant des fonctions carbamates.

Dans le cas de monoalcools, la molécule de bis-allophanate comprend 3 monomères isocyanates et 2 molécules alcool.

Les tris-allophanates sont définis de la même manière que les bis-allophanates.

Lorsque l'on fait la synthèse des allophanates à partir de plusieurs alcools comprenant des diols, il est préféré de commencer par la synthèse des bis-allophanates issus de diols pour éviter leur polycondensation.

En outre, selon l'invention, on peut également ajouter aux isocyanates polyfonctionnels tricondensats une combinaison d'homoallophanates différents, d'hétéroallophanates différents ou d'un mélange de ces deux catégories ou encore un mélange d'homoallophanates, et/ou d'hétéroallophanates obtenus avec des alcools différents.

Par composés condensats "lourds", on entend ceux obtenus par réaction de plus de quatre monomères les uns sur les autres.

Par allophanates lourds, on entend les produits allophanates n'entrant dans aucune des catégories précédemment définies.

En particulier, entrent dans la catégorie des allophanates lourds les allophanates comprenant une fonction isocyanate dérivée (biuret et/ou isocyanurate) et au moins une fonction allophanate et les composés comportant au moins trois fonctions allophanates encore désignées par le terme allophanates tricondensats.

La présente invention n'est pas limitée à la nature des isocyanates monomères mise en oeuvre. Ainsi, les isocyanates monomères peuvent être mono-, avantageusement di-, tri-, de préférence diisocyanates aliphatiques, y compris cycloaliphatiques et arylaliphatiques, tels que :

- les polyméthylènediisocyanates et notamment l'hexaméthylène diisocyanate, le 2-méthyl pentaméthylène diisocyanate, le 4-isocyanatométhyl octaméthylène diisocyanate, le 2,4,4-triméthyl hexaméthylène diisocyanate ;
- l'isophorone diisocyanate, le norbornane diisocyanate, le 1,3-bis(isocyanatométhyl)cyclohexane (BIC), le $H_{12}$-MDI et le cyclohexyl 1,4-diisocyanate ;
- les arylènedialcoylènediisocyanates (tel que $OCN-(CH_2)_p-\varnothing-(CH_2)_q-NCO$), p et q étant des nombres entiers identiques ou différents compris entre 1 et 6, de préférence 2 et 4;
- ou encore aromatiques tels que le toluylène diisocyanate.

Les isocyanates aromatiques et les isocyanates dont la fonction isocyanate est portée par un carbone néopentylique ne sont pas préférés.

Les isocyanates préférés visés par l'invention sont ceux dans lesquels au moins une, avantageusement deux, de préférence trois des conditions ci-après sont remplies.

- au moins une, avantageusement deux, des fonctions NCO sont reliées à un squelette hydrocarboné, par l'intermédiaire d'un carbone saturé ($sp^3$);
- au moins un, avantageusement deux, desdits carbones saturés ($sp^3$) est porteur d'au moins un, avantageusement deux hydrogène(s). En d'autres termes il a été trouvé que l'on obtenait de meilleurs résultats lorsque le carbone porteur de la fonction isocyanate était porteur d'un hydrogène, de préférence de deux hydrogènes) ; il est en outre même préférable que lesdits carbones saturés ($sp^3$) soit au moins pour le tiers, avantageusement au moins pour la moitié, de préférence au moins pour les deux tiers, reliés audit squelette par un atome de carbone lui-même porteur d'au moins un hydrogène, plus préférentiellement deux ;
- tous les carbones intermédiaires à partir desquels les fonctions isocyanates sont reliées au squelette hydrocarboné, sont des carbones saturés ($sp^3$), lesquels sont avantageusement en partie, de préférence en totalité, porteurs d'un hydrogène, de préférence de deux hydrogènes. Il est en outre même préférable que lesdits carbones saturés ($sp^3$) soit au moins pour le tiers, avantageusement au moins pour la moitié, de préférence au moins pour les deux tiers, reliés audit squelette par un atome de carbone lui-même porteur d'au moins un hydrogène, plus préférentiellement deux.

De manière générale, les isocyanates de départ (monomères) préférés sont ceux comportant au moins un enchaînement polyméthylène (comprenant de 2 à 6 chaînons méthylène).

On préfère les isocyanates, en particulier les diisocyanates aliphatiques, notamment les isocyanates d'alkyle en $C_1$-$C_{10}$ dans laquelle la chaîne alkyle est linéaire ou faiblement ramifiée. Par "faible ramification", on entend l'absence de carbone tertiaire et néopentylique.

L'HDI, l'IPDI, le NBDI, l'$H_{12}$-MDI et le MPDI sont particulièrement préférés.

De manière générale, les isocyanates aliphatiques présentant en règle générale une viscosité plus faible que les isocyanates cycloaliphatiques, on préférera éviter d'utiliser des allophanates isocyanates à fonctions cycloaliphatiques lorsque l'effet recherché est d'abaisser la viscosité de (poly)isocyanates isocyanurates obtenus à partir d'isocyanates à fonctions aliphatiques.

D'une manière générale, pour obtenir un abaissement du taux de viscosité relativement significatif, il faut que le mélange soit tel qu'il présente une viscosité plus faible que celle du mélange initial, exempt de monomères et de solvant, à savoir d'au moins environ 1/4, de préférence d'au moins environ 1/3, avantageusement d'au moins environ 2/5 par rapport à celle du mélange initial sans allophanates.

L'invention a plus particulièrement pour objet un procédé de préparation d'une composition d'isocyanates polyfonctionnels tricondensats, de viscosité abaissée, à partir d'isocyanates comprenant les étapes suivantes a) et b) dans un ordre indifférent :

a) (cyclo)condensation, en présence d'un catalyseur, d'un ou plusieurs premier(s) isocyanate(s) monomère(s) identiques ou différents jusqu'à obtention du taux de transformation recherché ;
b) réaction d'un ou plusieurs second(s) isocyanate(s) monomère(s) identique(s) ou différent(s) entre eux et identiques ou différents du(des) premier(s) isocyanate(s) monomère(s), avec un alcool alkylique à chaîne linéaire en $C_4$-$C_8$ pour former un carbamate, la réaction étant éventuellement catalysée, et réaction simultanée ou subséquente du carbamate avec un ou plusieurs isocyanate(s) monomères identiques ou différents des précédents pour obtenir un allophanate ou un mélange d'allophanates ; et les étapes c) et d) dans un ordre indifférent :
c) mélange du produit réactionnel de l'étape a) avec tout ou partie du produit réactionnel de l'étape b) ; et éventuellement
d) élimination des isocyanates monomères.

La réaction de (cyclo)condensation est avantageusement une réaction de (cyclo)trimérisation, laquelle est réalisée en présence d'un catalyseur de (cyclo)trimérisation connu en soi.

Lorsque l'on utilise un catalyseur pour la réaction de carbamatation, on utilisera avantageusement le même catalyseur pour la réaction d'allophanatation. Toutefois, on pourra utiliser des catalyseurs différents.

Les réactions de carbamatation et d'allophanatation peuvent être réalisées en deux temps, par exemple en augmentant la température du milieu réactionnel jusqu'à ce qu'ait lieu la réaction de carbamatation et en augmentant ultérieurement la température jusqu'à se produise la réaction d'allophanatation.

Les deux réactions peuvent également avoir lieu simultanément par augmentation de la température de réaction d'emblée jusqu'à la température d'allophanatation.

On peut utiliser dans les étapes a) et b) du procédé, le(s) même(s) isocyanate(s) monomère(s) qui sera(seront)

alors soumis parallèlement à une trimérisation catalytique et une réaction de carbamatation suivie d'une réaction d'allophanatation, les milieux réactionnels éventuellement purifiés étant ensuite mélangés jusqu'à obtention de la viscosité voulue.

On peut également, pour diminuer la viscosité d'un isocyanate polyfonctionnel tricondensat d'alkyle supérieur (comportant plus de 10 atomes de carbone), éventuellement ramifié, cycloalkyle ou aromatique donc de viscosité plus élevée que celle d'un polyisocyanate d'alkyle inférieur (comportant 10 atomes de carbone au plus), ajouter au produit de trimérisation le produit obtenu après carbamatation et allophanatation d'un ou plusieurs isocyanate(s) différent(s) du (des) premier(s) présentant une plus faible viscosité que celle qui serait obtenue par carbamatation puis allophanatation du(des) premier(s) isocyanate(s).

A cet effet, le(s) isocyanate(s)utilisés pour les réactions de carbamatation/allophanatation sera(seront) avantageusement un ou plusieurs isocyanate(s) d'alkyle linéaire, en particulier l'HDI.

L'étape a) est conduite dans les conditions habituelles de trimérisation catalytique d'isocyanates.

On peut citer, à titre d'exemple, pour les tricondensats à fonctions isocyanates la réaction classique, de l'HDI par catalyse en présence d'un dérivé aminosilylé notamment un silane ou un disilazane, de préférence d'hexaméthyldisilazane (HMDZ) tel que décrit dans EP 57 653 ou en présence d'un catalyseur d'ammonium quaternaire.

Les conditions réactionnelles comprennent notamment pour une réaction catalysée par l'HMDZ une quantité de catalyseur de l'ordre de 1.2 % en poids par rapport au poids de l'HDI, une durée de réaction d'environ 2 h 30 et une température d'environ 120°C.

Dans ces conditions, le taux de transformation des fonctions isocyanates est de 32,7 %, ce qui correspond à l'obtention d'un mélange (poly)isocyanate isocyanurate dont le taux de fonctions trimères vraies d'HDI (comprenant un cycle isocyanurate unique), est de l'ordre de 50 % en poids.

On peut également citer les réactions catalysées par les acides carboxyliques en présence d'eau pour l'obtention de condensats à motif(s) biuret(s) (brevet FR 86 12 524).

L'étape b) comprend une réaction de carbamatation classique suivie d'une réaction d'allophanatation classique, les deux réactions pouvant être catalysées par un même catalyseur ou une combinaison de catalyseurs, et les deux réactions pouvant se dérouler de manière simultanée dans un réacteur unique.

Dans un premier temps, l'(les) isocyanate(s) utilisé(s) pour la réaction d'allophanatation identique(s) ou différent (s) du(des) isocyanate(s) utilisé(s) pour la réaction de (cyclo)condensation, avantageusement (cyclo)trimérisation est (sont) mis à réagir éventuellement en présence d'un catalyseur d'allophanatation avec un ou plusieurs composé(s) comportant au moins une fonction alcool. La réaction est conduite à une température qui est avantageusement d'environ 80°C à environ 100°C, lorsque les réactions de carbamatation et d'allophanatation sont effectuées en deux temps ou directement à une température de l'ordre de 100°C à 180°C lorsque les réactions de carbamatation et d'allophanatation sont effectuées simultanément

On utilise les alcools alkyliques à chaîne linéaire en $C_4$-$C_8$.

La réaction est généralement poursuivie jusqu'à obtention d'un taux de NCO correspondant à la consommation d'au moins 80 % des fonctions alcools.

On peut également ajouter, lors de la réaction de carbamatation/allophanatation, un mélange de composés à fonction(s) alcool(s) différents.

Lorsque les réactions de carbamatation proprement dite et d'allophanatation sont dissociées, on peut après carbamatation, dans un second temps, élever la température du milieu réactionnel jusqu'à environ 100 à 180°C, de préférence aux alentours de 140°C pour l'HDI, pour réaliser la réaction d'allophanatation, celle-ci étant effectuée en présence éventuellement d'un catalyseur d'allophanatation, notamment un catalyseur à base d'étain, de zinc, ou autres métaux connus de l'homme du métier. On peut citer en particulier le dibutyl dilaurate d'étain (DBTL), le di(2-éthyl hexanoate) d'étain et le dichlorure d'étain, le DBTL étant préféré.

Les quantités de catalyseur sont avantageusement comprises entre 0,001 et 0,1 %, notamment 0,001 et 0,05%, de préférence d'environ 0.005 % en poids de métal, par rapport au poids d'isocyanate(s).

La durée de réaction est avantageusement de l'ordre de 1 à 24 heures, de préférence entre 3 et 7 heures.

La réaction d'allophanatation est conduite de façon à obtenir majoritairement des allophanates primaires, tels que définis précédemment

On peut utiliser un isocyanate différent de celui utilisé dans la réaction de carbamatation. On obtient alors un allophanate mixte.

Pour obtenir majoritairement des allophanates primaires, le rapport fonctions isocyanates (NCO)/fonctions hydroxyles (OH) est avantageusement élevé. On mettra en oeuvre de préférence un rapport supérieur à 4, encore mieux supérieur à 6, un rapport de l'ordre de 8 étant particulièrement intéressant.

Lorsque le rapport NCO/OH est bas, la viscosité du produit final est élevée en raison de la présence significativement plus élevée d'allophanates lourds tels que les oligomères bis-allophanates ou homologues supérieurs. Ainsi, la viscosité de l'allophanate de HDI et de butyle est multipliée par 4 lorsque le rapport NCO/OH passe de 8 à 4.

Les réactions de l'étape a) et de l'étape b) sont suivies en mesurant les titres de NCO.

L'étape c) est conduite en mélangeant les produits des étapes a) et b) en proportions variant en fonction de la viscosité finale souhaitée, conformément à la loi empirique donnée par la formule :

$$\text{Log } \eta_{\text{mélange}} = \text{Somme } x_i \text{ Log } \eta_i$$

avec η la viscosité du produit ou du mélange et x la fraction massique des produits du mélange.

Cette loi permet d'évaluer la teneur en allophanate(s) qu'il convient d'ajouter à une composition polyisocyanate à fluidifier en fonction de la viscosité que l'on souhaite obtenir.

Au cours de la présente invention on a pu démontrer de façon surprenante que les compositions à mélanger, quoique de structure et de masse moléculaire différentes, répondaient de manière qualitative ou semi-quantitative à cette loi.

En général, le(s) catalyseur(s) organométallique(s) est(sont) retrouvé(s) dans l'allophanate final. Les isocyanates monomères sont séparés des composés transformés par distillation ou par tout autre procédé de séparation (cristallisation, extraction par des gaz à l'état critique ou super critique... ) qui peut être réalisée séparément à l'issue des réactions a) et b) sur l'isocyanate polyfonctionnel tricondensat ou le mélange d'isocyanates polyfonctionnels tricondensats d'une part, et sur l'allophanate primaire ou le mélange d'allophanates dans les réacteurs correspondants ou après que le(s) isocyanate(s) polyfonctionnel(s) tricondensat(s) et allophanate(s) aient été mélangés, soit donc sur le mélange allophanate(s)/ isocyanate(s) polyfonctionnel(s) tricondensat(s).

A titre d'enseignement par l'exemple (paradigme), pour l'obtention de compositions comprenant un trimère d'HDI à motif isocyanurate (HDT), une viscosité de 700 mPa.s peut être obtenue en ajoutant à un produit de trimérisation de l'HDI obtenu par trimérisation avec un taux de transformation limité de l'HDI de l'ordre de 20 %, présentant une viscosité de l'ordre de 1200 mPa.s à 25°C, une quantité du produit d'allophanatation telle que la quantité d'allophanate primaire, de HDI et de butyle dans le produit final soit supérieure ou égale à environ 3 pour 100 g de mélange final, c'est-à-dire à environ 15% en poids d'allophanate primaire pour 100 g de mélange final.

Ainsi, lorsque l'on utilise de l'HDT ayant une viscosité de 2700 mPa.s à 25°C, on peut diminuer la viscosité à 1 200 mPa.s à 25°C en ajoutant une quantité d'allophanates de l'ordre de 25 % en poids par rapport au poids du mélange final pour obtenir une concentration finale d'environ 15 % en poids d'allophanate primaire.

L'étape d) est réalisée avantageusement par distillation sous vide de l'HDI dans les conditions habituelles.

Les compositions obtenues selon l'invention contiennent le(s) isocyanates polyfonctionnels tricondensats vrai (s) ainsi que des condensats lourds, obtenus par (cyclo)condensation catalytique de(s) l'isocyanate(s) monomère(s) de départ (premier(s) isocyanate(s) et éventuellement autres monomères présents), du monoallophanate primaire et des composés allophanates lourds tels que di-, tri-allophanates, du(des) deuxième(s) isocyanate(s) et éventuellement troisième(s) isocyanate(s) (dans le cas d'hétéroallophanates) et de l'alcool ou du mélange d'alcools employés pour la réaction de carbamatation, du bis-allophanate, du tris-allophanate et homologues supérieurs. La réaction d'allophanatation est menée de manière à ce que la quantité résiduelle de carbamates (produit intermédiaire non totalement transformé) soit faible (généralement inférieure à 20 %, avantageusement inférieure à 10 %, de préférence inférieure à 5 %, en poids).

D'une manière générale, le rapport :

$$\frac{\text{Fonctions carbamates issues de la ou des molécule(s) alcool utilisée(s) pour faire l'allophanate}}{\text{Fonctions allophanates issues de la ou des molécule(s) alcool utilisée(s) pour faire l'allophanate}}$$

est inférieur à 0,5, de préférence à 0,2 et avantageusement inférieure à 0,1.

Le procédé selon la présente invention convient particulièrement bien aux tricondensats comportant des motifs biurets engendrant en général des viscosités élevées. Toutefois, lorsqu'on utilise des tricondensats à base isocyanurate, on préfère que la quantité de composants comportant des motifs biurets ne soit pas élevée (inférieure à 50%, de préférence inférieure à 25%, avantageusement inférieure à 10%).

Toutefois, même quand la teneur en motifs biurets est comprise entre 0,5 et 5% en masse des motifs isocyanurates, l'on obtient toujours d'excellents résultats.

En l'absence de motifs biurets et surtout lorsque l'on utilise comme isocyanurates de départ les mélanges qualifiés de "basse viscosité", c'est-à-dire ayant une viscosité au plus égale à 1500 mPa.s, généralement 1300 mPa.s, à 25° C, il est préférable pour obtenir un abaissement significatif en ajoutant une faible proportion d'allophanates (environ 30% en masse au plus), de prendre des mélanges allophanates dont la viscosité est inférieure à 500 mPa.s, de préférence inférieure à 200 mPa.s.

La composition selon l'invention ne contient quasiment pas d'allophanates comportant des motifs tricondensats, notamment isocyanurate obtenu par cyclotrimérisation de l'isocyanate de départ. Elle comprend moins de 10 %, avantageusement moins de 8 %, encore plus avantageusement moins de 5 %, de préférence moins de 4 % et de manière plus préférée moins de 3 %, et de manière encore plus préférée moins de 2 % pouvant aller jusqu'à moins de 1 % , en poids d'allophanates de tricondensats par rapport au poids total de la composition.

D'une manière générale, les compositions sont caractérisées par une quantité de composés allophanates généralement supérieure à 5% en poids et par un rapport G défini ci-après élevé :

$$G = \frac{\text{Polyisocyanates tricondensat vrai, issus de la condensation de trois molécules d'isocyanates identiques ou différentes non modifiées carbamate, ni allophanate}}{\text{Somme des molécules polyisocyanates porteuses d'au moins une fonction tri condensat, issue de la condensation de trois molécules d'isocyanates identiques ou différentes.}}$$

(L'ensemble des molécules porteuses d'au moins une fonction tri condensat est formé par les composés polyisocyanates tricondensats vrais, les composés polyisocyanates tricondensats dont une fonction isocyanate au moins est engagée dans une fonction carbamate, ou une fonction allophanate ou une fonction hétérocyclique choisie parmi les fonctions urétidine dione, isocyanurate, etc...)

Le rapport G est généralement supérieur à 0,3, de préférence supérieur à 0,4, avantageusement supérieur à 0,5.

Une telle composition est nouvelle.

L'invention a donc également pour objet une composition d'isocyanates polyfonctionnels tricondensats, de viscosité abaissée, comprenant au moins un isocyanate polyfonctionnel tricondensat vrai et au moins un allophanate primaire préparé à partir d'alcools alkyliques à chaîne linéaire en $C_4$-$C_8$, ladite composition étant caractérisée en ce qu'elle comprend moins de 10 %, avantageusement moins de 8 %, encore plus avantageusement moins de 5 %, de préférence moins de 4 % et de manière plus préférée moins de 3 %, et de manière encore plus préférée moins de 2 % pouvant aller jusqu'à moins de 1 % d'allophanates de tricondensats par rapport au poids total de la composition.

L'invention a également pour objet une composition d'isocyanates polyfonctionnels tricondensats, de viscosité significativement abaissée, comprenant au moins un isocyanate polyfonctionnel tricondensat vrai et au moins un allophanate primaire préparé à partir d'alcools alkyliques à chaîne linéaire en $C_4$-$C_8$, ladite composition comprenant moins de 10% en poids d'allophanates de tricondensats par rapport au poids total de composition et répondant à au moins une des conditions ci-après:

- un rapport G généralement supérieur à 0,3, de préférence supérieur à 0,4, avantageusement supérieur à 0,5,
- un rapport pondéral allophanate primaire/allophanate primaire + trimère vrai compris entre 2,5 et 99 %, avantageusement compris entre 3 et 60% et de préférence entre 3,5 et 40 %,
- les tricondensats sont issus d'une réaction de tricondensation pour laquelle le taux de transformation du ou des monomères isocyanates identiques ou différents en polyisocyanates polyfonctionnels tricondensats contenus dans la composition est supérieur à 8%, de préférence 10%, avantageusement 15 %,
- il y a présence d'au moins 1% et d'au plus 99% de biuret, de préférence de 2% au moins et de 75% au plus, ces quantités étant données en poids.

Avantageusement, on préfère que les compositions d'isocyanates polyfonctionnels tricondensats, de viscosité abaissée, comprenant au moins un isocyanate polyfonctionnel tricondensat vrai et au moins un allophanate, répondent aux deux premières conditions, voire aux trois premières conditions et mieux encore, aux quatre conditions ci-dessus.

Pour obtenir des compositions de basse viscosité comprenant des isocyanates polyfonctionnels tricondensats à partir d'isocyanates cycloaliphatiques, on peut procéder de la même manière que décrite ci-dessus et éventuellement ajouter une quantité faible de solvant (en général moins de 1/3, avantageusement moins de 1/4, de préférence moins de 1/10. en poids par rapport au poids total de la composition.

Les compositions obtenues selon l'invention peuvent être sous forme de poudres et fournir une viscosité abaissée lors du passage à l'état fondu par rapport aux produits ne comportant pas d'allophanates primaires.

Les compositions sous leurs différentes formulations, solvantées aqueuses ou hydroorganiques ou sous forme poudres, peuvent également comporter des groupes protecteurs des fonctions isocyanates identiques ou différents. Les fonctions isocyanates peuvent être protégées partiellement ou en totalité. Le rapport fonctions isocyanates libres

sur fonctions isocyanates masquées est choisi par l'homme de l'art en fonction de l'application visée.

Les compositions obtenues selon l'invention peuvent être utilisées en formulations aqueuses avec éventuellement ajout d'additifs de formulations tels que des tensioactifs, ioniques ou non ioniques, ou greffage de manière réversible ou irréversible sur les fonctions isocyanates de composés polyoxyalkylènes divers tels que des dérivés de polyéthylène glycols ou des amines polyoxyéthylénées.

Ces compositions de polyisocyanates, à fonctions isocyanates éventuellement masquées en partiel ou en totalité peuvent également conduire à des émulsions ou suspensions telles que décrit dans FR 2 703 357 et EP 691 993.

Les polyols peuvent en outre servir d'agents de formulations de ces compositions polyisocyanates pour faire des solutions aqueuses, des émulsions ou des dispersions.

De même, ces compositions peuvent être utilisées pour préparer des compositions polyuréthannes poudres ou solvantées ou en solution aqueuse ou hydroorganique éventuellement masquées par des agents de masquage temporaires et/ou permanents. Le choix du polyol est alors dépendant de l'application visée.

Les compositions objets de la présente invention sont utilisées avec des additifs classiques des revêtements, à savoir agents de mouillage, pigments, agents d'étalement. agents de mar-résistance et tout autre composé connu de l'homme de l'art utilisé dans les applications ci-dessus mentionnées.

Parmi les nombreux avantages que présente l'invention, on peut citer outre la viscosité abaissée le fait que le procédé selon l'invention permet de régler la viscosité de manière rapide et aisée par ajustement de la quantité de l'un ou l'autre des composants (isocyanates polyfonctionnels tricondensats ou allophanate(s)) du mélange sans être tenu de recourir à une synthèse totale, à partir des monomères de départ et de l'alcool.

En outre, pour un même rendement en allophanates par rapport au procédé de l'état de la technique, le taux de transformation, du monomère pour une viscosité donnée est significativement supérieur.

Les exemples ci-après illustrent l'invention.

Le titre NCO est exprimé soit en % de NCO pour 100 g de mélange, soit en mole de NCO pour 100 g de mélange.

**EXEMPLE 1** : Préparation d'HDT basse viscosité soit "LV".

Dans un réacteur tricol de 6 litres on introduit 4584 g d'hexaméthylène diisocyanate (HDI). On chauffe et agite le milieu réactionnel. On additionne, à 113° C, 27,5 g d'hexaméthyle disilazane (HMDZ). La température du milieu réactionnel est alors montée à 120° C. La température est maintenue pendant 2 heure 15 minutes. Le titre NCO mesuré à ce stade est de 1,069 mole de NCO pour 100 g de mélange, ce qui donne un taux de transformation de l'HDI de 19,3 %. La température du milieu réactionnel est descendue à 80° C et 16 ml de n-butanol est ajouté pour bloquer la réaction de trimérisation. Après une heure de réaction l'HDI monomère est éliminé par évaporation sous vide pour obtenir un produit HDT qui a une viscosité de 1260 mPa.s à 25° C, un taux de HDI résiduel inférieur à 0,15% et une coloration de 5 hazen.

**EXEMPLE 2 :** Préparation d'allophanate d'HDI et de butyle de très basse viscosité.

Dans un réacteur de 6 l, on introduit 4830 g de HDI. Sous agitation on coule en 50 minutes 532 g de n-butanol tout en laissant monter la température de réaction à 108° C. 1,3 g de dibutyl dilaurate d'étain est ajouté à 125° C et la température est montée à 140° C. Après 5 heures de réaction à 140° C environ, on arrête la réaction par refroidissement. Le titre NCO est de 0.786 mole de NCO pour 100 g de mélange. L'HDI monomère est éliminé par évaporation sous vide pour obtenir un allophanate de HDI et de n-butyle de viscosité égale à 140 cps et de titre 0,405 mole de NCO pour 100 g de produit, soit 17% en poids de NCO pour 100 g de mélange. Le rendement récupéré en produit fini est de 50% en poids environ. La quantité de HDI résiduel est de 0,05%. La quantité d'allophanates primaires vrais de HDI et de n-butyle est de 57,8% en poids. La coloration du produit est de 10-15 hazen.

**EXEMPLE 3** : Allophanate de HDI et de butyle de moyenne viscosité.

On opère de la même manière que pour l'exemple 2 mais avec un rapport NCO/OH de 4.

Le produit présente une viscosité de 600 mPa.s à 25° C, un titre NCO de 0,303 soit 12,7%, et un titre en HDI monomère résiduel de 0,1%. Le rendement récupéré en produit fini est de 76,6% en poids. La composition du produit obtenu est la suivante en poids :

- allophanate primaire (mono-allophanate)
  de HDI et de n-butyle : 30,6 %.
- monocarbamate de butyle : 1 %
- bis-allophanate :25,7%
- tris-allophanate :18,0%

- lourds : 24,7 %.

La coloration du produit est de 10-15 hazen.

**EXEMPLE 4 :** Préparation du mélange basse viscosité.

Le mélange a été réalisé en appliquant la loi de viscosité précédemment donnée.
On a réalisé un mélange contenant 75 % en poids de la composition obtenue à l'exemple 1 avec 25 % en poids de la composition obtenue à l'exemple 2.
Les caractéristiques des produits sont obtenues à partir d'analyses quantifiées par infrarouge de produits séparés sur colonne séparative.
Les caractéristiques du mélange sont indiqués dans le tableau ci-dessous.

Tableau : Caractéristiques d'un mélange HDT allophanate d'HDI et de butyle.

| | Produit de l'exemple 1 | Produit de l'exemple 2 | Caractéristiques des mélanges | |
|---|---|---|---|---|
| % d'allophanate primaire de HDI et de n-butyle | 0.7% | 56.8% | 14.45 | 14.45 |
| %(cyclo) tricondensat vrai | 61% | 0% | 45.75 | 45 |
| Rapport G (cyclo) tricondensat vrai/somme des (cyclo) tricondensats | 0.69 | 0 | 0.69 | 0.68 |
| Allophanate primaire/ Allophanate primaire + (cyclo) tricondensat vrai | 1 % | | 24 % | 23.8 % |
| Fonctions Biuret | 6 % | | 4.5 % | 4.5 % |
| viscosité (mPa.s à 25°C (test à la chute de bille) | 1260 | 600 | 720 | 730 |

Le gain en viscosité exprimé par le rapport :

(viscosité du produit exemple 1 – viscosité du mélange)/viscosité du produit exemple 1 est égal à 42,9 %.

**EXEMPLE 5 :** Viscosités de mélanges selon l'invention en fonction de la teneur en allophanates.

On a mélangé en proportions variables de l'HDT obtenu selon l'exemple 1 avec des allophanates obtenus selon l'exemple 2.
Les viscosités sont reportées dans la figure annexée.

**EXEMPLE 6 :** Viscosités de mélanges HDB/allophanate de HDI et de butyle.

On a préparé des mélanges hexaméthylène diisocyanate biuret (HDB)/allophanate de HDI et de butyle comme décrit ci-dessus en proportions variables et mesuré leur viscosité.
Les résultats sont reportés dans le tableau ci-après :

Tableau : Caractéristiques d'un mélange HDB allophanate d'HDI et de butyle.

| HDB (% en poids) | Allophanate HDI/butyle (% en poids) | Viscosité (mPa.s. 25° C) | |
|---|---|---|---|
| | | (1) | (2) |
| 100 | 0 | 10 500 | 17 100 |
| 90 | 10 | 6200 | 10 250 |

(suite)

| HDB (% en poids) | Allophanate HDI/butyle (% en poids) | Viscosité (mPa.s. 25° C) | |
|---|---|---|---|
| | | (1) | (2) |
| 75 | 25 | 3 300 | 4600 |
| 50 | 50 | 750 | 1450 |
| (1) HDB de viscosité standard (TT monomères ≅ 40 %)<br>(2) HDB de viscosité élevée | | | |

**EXEMPLE 7** : Synthèse d'un trimère de norbornane diisocyanate trimère de basse viscosité (NBDT LV)

Le produit est synthétisé selon le même protocole que celui décrit pour l'exemple 1, en utilisant 0,75 g d'hexaméthyle disilazane pour 100 g de norbornane diisocyanate (NBDI) et un temps de réaction de 3 heures à 120° C. Le taux de transformation du NBDI avant arrêt de la réaction est de 20 %.

Le produit purifié à l'excès de monomère par distillation est un solide qui a une viscosité mesurée de 4070 mPa.s à 25° C et à 80% d'extrait sec dans l'acétate de n-butyle et de 252 mPa.s à 25° C et à 70% d'extrait sec dans l'acétate de n-butyle

**EXEMPLE 8 :** Synthèse d'un trimère de norbornane diisocyanate trimère (NBDT)

Le produit est synthétisé selon le même protocole que celui décrit pour l'exemple 1, en utilisant 1,4 g d'hexaméthyle disilazane pour 100 g de norbornane diisocyanate (NBDI) et un temps de réaction de 3 heures à 120° C. Le taux de transformation du NBDI avant arrêt de la réaction est de 26,4%. Le produit purifié de l'excès de monomère par distillation est un solide qui a une viscosité mesurée de 13400 mPa.s à 25° C et à 80% d'extrait sec dans l'acétate de n-butyle.

**EXEMPLE 9 :** Préparation de compositions NBDT/allophanate de HDI et de n-butyle de basse viscosité.

Les compositions ont été préparées comme indiqué au tableau ci-après :

| | Produit de l'exemple 8 | Produit de l'exemple 9 | Produit de l'exemple 2 | Caractéristiques des mélanges | |
|---|---|---|---|---|---|
| % composition en poids | | | | 60/40 exemple 2/ exemple 8 | 60/40 exemple 2/ exemple 9 |
| TT en HDI | | | 50% | | |
| TT en NBDI | 20 % | 26.4% | | | |
| HDI résiduel | | | 0.05 | 0.03 | 0.03 |
| NBDI résiduel | 0.61 | | | 0.25 | |
| Viscosité en mPa.s à 25° C et à 100 % d'extrait sec | solide* | solide** | 140 | 4100 | 4500 |
| Coloration en Hazen | 10-15 | 10-15 | 10-15 | 10-15 | 10-15 |
| % d'allophanate primaire de HDI et de n-butyle | | | 56.8% | 34 | 34 |
| % (cyclo) tricondensat vrai | 74% | 70 % | 0 % | 29.6 | 28 |

(suite)

| | Produit de l'exemple 8 | Produit de l'exemple 9 | Produit de l'exemple 2 | Caractéristiques des mélanges | |
|---|---|---|---|---|---|
| % composition en poids | | | | 60/40 exemple 2/ exemple 8 | 60/40 exemple 2/ exemple 9 |
| Rapport G (cyclo) tricondensat vrai/somme des (cyclo) tricondensats | 0.74 | 0.7 | 0 | 0.74 | 0.7 |
| Allophanate primaire/ Allophanate primaire + (cyclo) tricondensat vrai | | | | 53.5 | 55 |
| Gain en viscosité | | | | > 50 % | > 50 % |
| • * 4070 mPa.s à 25° C et à 80% d'extrait sec dans l'acétate de n-butyle<br>• ** 13400 mPa.s à 25° C et à 80 % d'extrait sec dans l'acétate de n-butyle. | | | | | |

Les gains en viscosité sont largement supérieurs à 50 % compte tenu du fait que les produits sont des solides et que la viscosité mesurée à 80 % d'extrait sec est équivalente voire supérieure à la viscosité des deux compositions.

**EXEMPLE 10 :** Préparation de compositions NBDT/HDT et allophanate de HDI et de n-butyle de basse viscosité

Les compositions ont été préparées comme indiqué au tableau ci-après :

| | Produit de l'exemple 8 | Produit de l'exemple 1 | Produit de l'exemple 2 | Caractéristiques des mélanges |
|---|---|---|---|---|
| % composition en poids | | | | 45/15/40 Pdt1/Pdt2/Pdt 8 |
| TT en HDI | | 19.3 | 50% | |
| TT en NBDI | 20 % | | | |
| HDI résiduel | | 0.15 | 0.05 | 0.07 |
| NBDI résiduel | 0.61 | | | 0.25 |
| Viscosité en mPa.s à 25° C et à 100 % d'extrait sec | Solide* | 1260 | 140 | 18500 |
| Coloration en Hazen | 10-15 | 5 | 10-15 | 10-15 |
| % d'allophanate primaire de HDI et de n-butyle | | 0,7 | 56.8% | 8,7 |
| % (cycle) tricondensat vrai | 74% | 61 | 0% | 57 |

(suite)

| | Produit de l'exemple 8 | Produit de l'exemple 1 | Produit de l'exemple 2 | Caractéristiques des mélanges |
|---|---|---|---|---|
| % composition en poids | | | | 45/15/40 Pdt1/Pdt2/Pdt 8 |
| Rapport G (cyclo) tricondensat vrai/ somme des (cyclo) tricondensats | 0.74 | 0.69 | 0 | 0.72 |
| Allophanate primaire / allophanate primaire + (cyclo) tricondensat vrai | | | | 13 |
| Biuret | | 6 % | | 2.5 % |
| Gain en viscosité | | | | 34 % |
| • * 4070 mPa.s à 25° C et à 80 % d'extrait sec dans l'acétate de n-butyle<br>• ** 13400 mPa.s à 25° C et a 80 % d'extrait sec dans l'acétate de n-butyle. | | | | |

Le mélange des produits de l'exemple 8 et 1 dans un rapport 52/48 en poids présente une viscosité de l'ordre de 28000 mPa.s à 25° C à 100 % d'extrait sec.

Les gains en viscosité sont largement supérieurs à 50 % compte tenu du fait que les produits sont des solides et que la viscosité mesurée à 80 % d'extrait sec est équivalente voire supérieure à la viscosité des deux compositions.

## Revendications

1. Procédé de préparation d'une composition d'isocyanates polyfonctionnels tricondensats, de préférence comprenant au moins un groupe isocyanurate et/ou biuret, consistant à ajouter à un isocyanate polyfonctionnel tricondensat, ou à un mélange de différents isocyanates polyfonctionnels tricondensats, obtenus par (cyclo)condensation, notamment (cyclo)trimérisation d'un ou plusieurs isocyanates monomères identiques ou différents et éventuellement d'un autre monomère, un allophanate d'un ou plusieurs isocyanates identiques ou différents, ou un mélange de différents allophanates, les isocyanates ou mélanges d'isocyanates monomères utilisés pour la préparation du(des) isocyanate(s) polyfonctionnel(s) étant identiques ou différents du(des) isocyanate(s) ou du mélange d'isocyanates utilisés pour la préparation du(des) allophanate(s) et le(s) allophanate(s) est(sont) préparé(s) à partir d'alcools alkyliques à chaîne linéaire en $C_4$-$C_8$.

2. Procédé selon la revendication 1, **caractérisé en ce que** les isocyanates polyfonctionnels tricondensats répondent à la formule générale suivante :

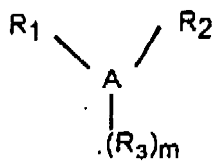

(I)

dans laquelle A représente

- un groupe isocyanurate de formule

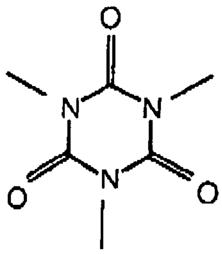 ;

- un de ses dérivés tels que les imino oxadiazine diones de formule suivante

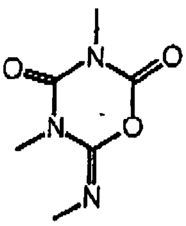

- un de ses dérivés tels que les oxadiazine triones de formule suivante

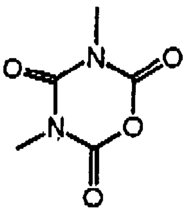

- un groupe biuret de formule

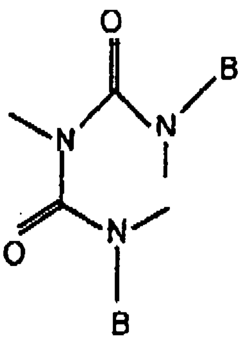

B étant H ou un groupe hydrocarboné, c'est-à-dire contenant du carbone et de l'hydrogène ainsi qu'éventuellement d'autres atomes (O, S, Si, ..) ayant de préférence 1 à 20 atomes de carbone; ou
- un groupe de formule:

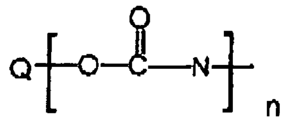

et dans laquelle $R_1$, $R_2$, $R_3$ identiques ou différents représentent un groupe hydrocarboné, notamment aliphatique, cycloaliphatique, hétérocyclique ou aromatique, comprenant une fonction isocyanate vraie ou dérivée,
Q est un groupe hydrocarboné, de préférence alcoyle, tel que défini pour $R_1$ à $R_3$.
m est un nombre entier de 0 à 2,
n est un nombre entier de 3 à 4.

3. Procédé selon l'une quelconque des revendications précédentes, dans lequel la composition d'isocyanates polyfonctionnels tricondensats comprend au moins un polyisocyanate isocyanurate vrai.

4. Procédé selon l'une quelconque des revendications précédentes, dans lequel les allophanates répondent à la formule générale II :

$$R_4 - NC(O)O - R_5 \quad \text{(II)}$$
$$|$$
$$CO - NHR_6$$

dans laquelle :

- $R_4$ et $R_6$ identiques ou différents représentent un groupe hydrocarboné, notamment aliphatique, cycloaliphatique, hétérocyclique ou aromatique, comprenant une fonction isocyanate vraie ou dérivée.
- $R_5$ représentant un groupe alcoyle, à savoir le reste d'un composé alcool après enlèvement de la fonction OH, à chaîne linéaire en $C_4$-$C_8$.

5. Procédé selon l'une quelconque des revendications précédentes, **caractérisé en ce que** l'on ajoute aux isocyanates polyfonctionnels tricondensats un mélange d'allophananates comprenant un allophanate primaire, de préférence contenant environ 1/4, avantageusement environ 1/3, de préférence environ 2/5 (en masse) d'allophanate(s) primaire (s).

6. Procédé selon l'une quelconque des revendications précédentes, **caractérisé en ce que** le mélange d'allophanates comprend des mono-, bis- et tris-allophanates, en une quantité avantageusement d'au moins 2/3, de préférence d'au moins 75 %, et de manière encore plus préférée d'au moins 90 % en poids par rapport au poids total de la composition d'allophanates après élimination des monomères n'ayant pas réagi.

7. Procédé selon l'une quelconque des revendications précédentes, **caractérisé en ce que** la quantité de bis-allophanate représente jusqu'à 10 %, voire jusqu'à 20 % du poids total de la composition d'allophanates.

8. Procédé selon l'une quelconque des revendications précédentes, **caractérisé en ce que** la quantité de tris-allophanates est inférieure ou égale à 30 %, avantageusement 20 %, de préférence 15 % en poids par rapport au poids total de la composition totale de la composition.

9. Procédé selon l'une quelconque des revendications précédentes, **caractérisé en ce que** le rapport

$$\frac{\text{fonctions bis-allophanates + fonctions tris-allophanates}}{\text{fonctions mono-allophanates}} \text{ est égal ou supérieur à 0,1.}$$

et peut aller jusqu'à 0,3 voire jusqu'à 0,5.

**10.** Procédé de préparation selon la revendication 1 d'une composition d'isocyanates polyfonctionnels tricondensats de faible viscosité comprenant des groupes isocyanurates et/ou biurets, à partir d'isocyanates comprenant les étapes suivantes a) et b) dans un ordre indifférent:

a) (cyclo)condensation catalytique d'un ou plusieurs premier(s) isocyanate(s) monomère(s) identiques ou différents et éventuellement d'un ou plusieurs monomères identiques ou différents jusqu'à obtention du taux de transformation recherché;
b) réaction d'un ou plusieurs second(s) isocyanate(s) monomère(s) identique(s) ou différent(s) entre eux et identiques ou différents du(des) premier(s) isocyanate(s) monomère(s), avec un alcool alkylique à chaîne linéaire en $C_4$-$C_8$, pour former un carbamate, la réaction étant éventuellement catalysée, et réaction simultanée ou subséquente du carbamate avec un ou plusieurs troisièmes isocyanate(s) monomères identiques ou différents entre eux et identiques ou différents des précédents pour obtenir un allophanate ou un mélange d'allophanates ;

et les étapes c) et d) dans un ordre indifférent :

c) mélange du produit réactionnel de l'étape a) avec tout ou partie du produit réactionnel de l'étape b) ; et éventuellement
d) élimination des (isocyanates) monomères.

**11.** Procédé selon la revendication 1 ou 2 **caractérisé en ce que** le(s) isocyanate(s) utilisés pour la réaction de (cyclo) condensation est(sont) identique(s) à(aux) isocyanate(s) utilisés pour la réaction d'allophanatation.

**12.** Procédé selon la revendication 1 ou 2, **caractérisé en ce que** le(s) isocyanate(s) utilisé(s) pour la réaction de (cyclo) condensation est(sont) différent(s) du(des) isocyanates(s) utilisé(s) pour la réaction d'allophanatation.

**13.** Procédé selon l'une des revendications précédentes, **caractérisé en ce que** le(s) isocyanate(s) utilisés pour la réaction d'allophanatation et le(s) isocyanate(s) utilisés pour la réaction de cyclotrimérisation répondent à au moins une, avantageusement deux, de préférence trois des conditions ci-après ;

- au moins une, avantageusement deux, des fonctions NCO sont reliées à un squelette hydrocarboné, par l'intermédiaire d'un carbone saturé ($sp^3$) ;
- au moins un, avantageusement deux, desdits carbones saturés ($sp^3$) est porteur d'au moins un, avantageusement deux hydrogène(s).
- tous les carbones intermédiaires à partir desquels les fonctions isocyanates sont reliées au squelette hydrocarboné, sont des carbones saturés ($sp^3$), lesquels sont avantageusement en partie, de préférence en totalité, porteurs d'un hydrogène, de préférence de deux hydrogènes.

**14.** Procédé selon l'une quelconque des revendications 10 à 13, **caractérisé en ce que** le rapport NCO/OH dans l'étape b) est supérieur à 4, de préférence supérieur à 6.

**15.** Procédé selon l'une quelconque des revendications précédentes, **caractérisé en ce que** l'on mélange au moins environ 25 % en poids du produit de l'étape b) avec le produit de l'étape a) qui est de l'HDT (Hexaméthylène Diisocyanate Trimère).

**16.** Composition d'isocyanates polyfonctionnels tricondensats, de viscosité abaissée, comprenant au moins un isocyanate polyfonctionnel tricondensat vrai et au moins un allophanate primaire préparé à partir d'un alcool alkylique à chaîne linéaire en $C_4$-$C_8$, ladite composition étant **caractérisée en ce qu'**elle comprend moins de 10 %, avantageusement moins de 8 %, encore plus avantageusement moins de 5 %. de préférence moins de 4 % et de manière plus préférée moins de 3 %, et de manière encore plus préférée moins de 2 % pouvant aller jusqu'à moins de 1 % d'allophanates de tricondensats par rapport au poids total de la composition.

**17.** Composition selon la revendication 16, comprenant au moins un isocyanate polyfonctionnel tricondensat vrai et au moins un allophanate primaire préparé à partir d'un alcool alkylique à chaîne linéaire en $C_4$-$C_{8,}$ ladite composition répondant à au moins une, avantageusement au moins deux, plus avantageusement trois et de préférence quatre, des conditions ci-après :

- un rapport G défini par :

$$G = \frac{\text{Polyisocyanates tricondensat vrai, issus de la condensation de trois molécules d'isocyanates identiques ou différentes non modifiées carbamate, ni allophanate}}{\text{Somme des molécules polyisocyanates porteuses d'au moins une fonction tri condensat, issue de la condensation de trois molécules d'isocyanates identiques ou différentes.}}$$

généralement supérieur à 0,3, de préférence supérieur à 0,4, avantageusement supérieur à 0,5,
- un rapport pondéral allophanate /allophanate + trimère vrai compris entre 2,5 et 99 %, avantageusement compris entre 3 et 60% et de préférence entre 3,5 et 40 %,
- les tricondensats sont issus d'une réaction de tricondensation pour laquelle le taux ,de transformation du ou des monomères isocyanates identiques ou différents en polyisocyanates polyfonctionnels tricondensats contenus dans la composition est supérieur à 8%, de préférence 10%, avantageusement 15 %,
- il y a présence d'au moins 1% et d'au plus 99% de biuret, de préférence de 2% au moins et de 75% au plus, ces quantités étant données en poids.

**18.** Composition d'isocyanates polyfonctionnels tricondensats selon l'une des revendications 16 à 17, **caractérisée en ce que** l'allophanate comprend des mono-, bis- et tris-allophanates, en une quantité avantageusement d'au moins 2/3, de préférence d'au moins 75 %, et de manière encore plus préférée d'au moins 90 % en poids par rapport au poids total de la composition d'allophanates après élimination des monomères n'ayant pas réagi.

**19.** Composition d'isocyanates polyfonctionnels tricondensats selon l'une des revendications 16 à 18, **caractérisée en ce que** la quantité de bis-allophanate représente jusqu'à 10 %, voire jusqu'à 20 % du poids total de la composition d'allophanates.

**20.** Composition d'isocyanates polyfonctionnels tricondensats selon l'une des revendications 16 à 19, **caractérisée en ce que** la quantité de tris-allophanates est inférieure ou égale à 30 %, avantageusement 20 %, de préférence 15 % en poids par rapport au poids total de la composition totale de la composition.

**21.** Composition d'isocyanates polyfonctionnels tricondensats selon l'une des revendications 16 à 20, **caractérisée en ce que** le rapport

$$\frac{\text{fonctions bis-allophanates + fonctions tris-allophanates}}{\text{fonctions mono-allophanates}} \text{ est égal ou supérieur à 0,1,}$$

et peut aller jusqu'à 0,3 voire jusqu'à 0,5.

**22.** Composition d'isocyanates polyfonctionnels tricondensats selon la revendication 16 comportant de l'hexaméthylène diisocyanate biuret.

**23.** Composition selon l'une des revendications 16 à 22, **caractérisé en ce que** le(s) isocyanate(s) utilisé(s) pour la réaction de (cyclo) condensation est(sont) différent(s) du(des) isocyanate(s) utilisé(s) pour la réaction d'allophanatation.

**24.** Utilisation d'une composition selon l'une quelconque des revendications 16 à 23 pour la réalisation d'un revêtement.

## Claims

**1.** Process for preparing a polyfunctional isocyanate tri-condensation product, preferably comprising at least one isocyanurate and/or biuret group, comprising adding an allophanate of one or more identical or different isocyanates or a mixture of different allophanates to a polyfunctional isocyanate tri-condensate or a mixture of different polyfunctional isocyanate tri-condensates obtained by (cyclo)condensation, in particular (cyclo)trimerisation of one or more identical or different isocyanate monomers and possibly another monomer, the isocyanates or mixtures of isocyanate monomers used to prepare the polyfunctional isocyanate(s) (s) being the same as or different from the isocyanate(s) or mixture of isocyanates used to prepare the said allophanate(s), and the allophanate(s) is(are) prepared from straight-chain $C_4$-$C_8$ alkyl alcohols.

**2.** Process according to claim 1, **characterised in that** the polyfunctional isocyanate tri-condensates have the following general formula:

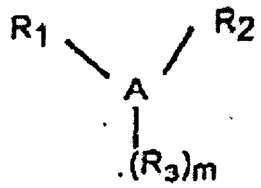

(I)

in which A represents

- an isocyanurate group of formula

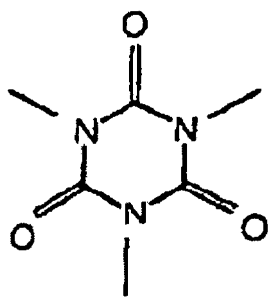

- one of its derivatives such as imino oxadiazine diones having the following formula

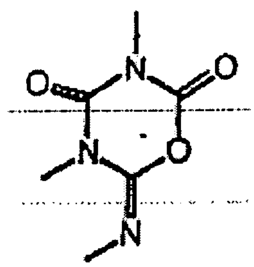

- one of its derivatives such as oxadiazine triones having the following formula

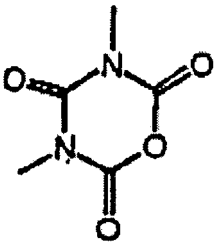

- a biuret group of formula

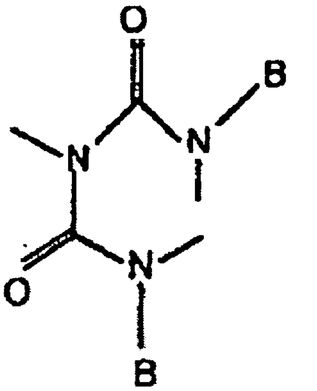

B being H or a hydrocarbon group, i.e. containing carbon and hydrogen as well as possibly other atoms (O, S, Si, etc.) having preferably 1 to 20 carbon atoms, or
- a group having the formula:

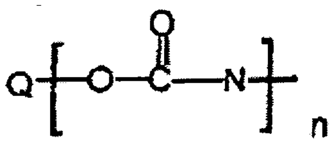

in which $R_1$, $R_2$, $R_3$, which are the same or different, represent a hydrocarbon group, in particular an aliphatic, cycloaliphatic, heterocyclic or aromatic group, comprising a true isocyanate group or derivative,
Q is a hydrocarbon group, preferably alkoyl, as defined for $R_1$ to $R_3$,
m is a whole number from 0 to 2,
n is a whole number from 3 to 4.

**3.** Process according to either of the preceding claims in which the polyfunctional isocyanate tri-condensation product comprises at least one true isocyanurate polyisocyanate.

**4.** Process according to any one of the preceding claims, in which the allophanates have the general formula II:

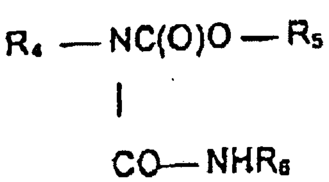

(II)

in which:

- $R_4$ and $R_6$, which are the same or different, represent a hydrocarbon group, in particular an aliphatic, cycloaliphatic, heterocyclic or aromatic group comprising a true isocyanate group or derivative,
- $R_5$ represents an alkoyl group, namely a residue of an alcohol compound after removal of the OH group, having a straight $C_4$-$C_8$ chain.

**5.** Process according to any one of the preceding claims, **characterised in that** an allophanate mixture comprising a primary allophanate, preferably containing approximately 1/4, advantageously approximately 1/3, and preferably approximately 2/5 (by mass) of primary allophanate(s) is added to the polyfunctional isocyanate tri-condensates .

**6.** Process according to any one of the preceding claims, **characterised in that** the allophanate mixture comprises mono-, bis- and tris-allophanates, in a quantity of advantageously at least 2/3, preferably at least 75% and even more preferably at least 90% by weight with respect to the total weight of the allophanate composition after removal of the unreacted monomers.

**7.** Process according to any one of the preceding claims, **characterised in that** the quantity of bis-allophanate represents up to 10%, or even up to 20%, of the total weight of the allophanate composition.

**8.** Process according to any one of the preceding claims, **characterised in that** the quantity of tris-allophanates is less than or equal to 30%, advantageously 20% and preferably 15% by weight in relation to the total weight of the total composition of the composition.

**9.** Process according to any one of the preceding claims, **characterised in that** the ratio

$$\frac{\text{bis-allophanate groups + tris-allophanate groups}}{\text{mono-allophanate groups}}$$

is equal to or greater than 0.1, or may be up to 0.3 or even up to 0.5.

**10.** Process of preparation according to claim 1 of a low viscosity polyfunctional isocyanate tri-condensation product comprising isocyanurate and/or biuret groups from isocyanates comprising the following stages a) and b) in any order:

a) catalytic (cyclo)condensation of one or more first identical or different isocyanate monomers and if appropriate one or more identical or different monomers until the level of conversion desired is achieved,
b) reacting one or more second identical or different isocyanate monomers which are the same as or different from the first isocyanate monomer(s) with a straight chain $C_4$-$C_8$ alkyl alcohol to form a carbamate, the reaction being catalysed if necessary, and simultaneous or subsequent reaction of the carbamate with one or more third identical or different isocyanate monomers which are the same as or different from the above in order to obtain an allophanate or a mixture of allophanates,

and stages c) and d) in any order:

c) mixing the reaction product from stage a) with all or part of the reaction product from stage b), and if appropriate
d) removing the (isocyanate) monomers.

**11.** Process according to claim 1 or 2, **characterised in that** the isocyanate(s) used for the (cyclo)condensation reaction is (are) identical to the isocyanate(s) used for the allophanation reaction.

**12.** Process according to claim 1 or 2, **characterised in that** the isocyanate(s) used for the (cyclo)condensation reaction is(are) different from the isocyanate(s) used for the allophanation reaction.

**13.** Process according to any one of the preceding claims, **characterised in that** the isocyanate(s) used for the allophanation reaction and the isocyanate(s) used for the cyclotrimerisation reaction fulfil at least one, advantageously two and preferably three of the following conditions:

- at least one and advantageously two of the NCO groups are connected to a hydrocarbon skeleton through a saturated carbon ($sp^3$),
- at least one and advantageously two of the said saturated carbons ($sp^3$) carries at least one and advantageously two hydrogens,
- all the intermediate carbons through which the isocyanate groups are connected to the hydrocarbon skeleton are saturated carbons ($sp^3$), some or preferably all of which advantageously carry a hydrogen, preferably two hydrogens.

**14.** Process according to any one of claims 10 to 13, **characterised in that** the NCO/OH ratio in stage b) is higher than 4 and preferably higher than 6.

**15.** Process according to any one of the preceding claims, **characterised in that** at least approximately 25% by weight of the product from stage b) is mixed with the product from stage a), which is HDT (hexamethylene diisocyanate trimer).

**16.** Low viscosity polyfunctional isocyanate tri-condensation product comprising at least one true polyfunctional isocyanate tri-condensate and at least one primary allophanate prepared from a straight chain $C_4$-$C_8$ alkyl alcohol, the said composition being **characterised in that** it comprises less than 10%, advantageously less than 8%, even more advantageously less than 5%, preferably less than 4% and more preferably less than 3%, and even more preferably less than 2%, which may be even less than 1%, of allophanate tri-condensate in relation to the total weight of the composition.

**17.** Composition according to claim 16, comprising at least one true polyfunctional isocyanate tri-condensate and at least one primary allophanate prepared from a straight chain $C_4$-$C_8$ alkyl alcohol, the said composition complying with at least one, advantageously at least two, more advantageously three and preferably four of the conditions below:

- a ratio G defined by:

$$G = \frac{\text{True polyisocyanate tri-condensates produced by the condensation of three identical or different isocyanate molecules which are not modified carbamate or allophanate}}{\text{Sum of the polyisocyanate molecules having at least one tri-condensate unit obtained from the condensation of three identical or different isocyanate molecules}}$$

of generally over 0.3, preferably over 0.4 advantageously over 0.5,
- an allophanate/allophanate + true trimer ratio of between 2.5 and 99%, advantageously between 3 and 60% and preferably between 3.5 and 40%,
- the trimer condensates have been produced by a trimer condensation reaction in which the level of conversion of the identical or different isocyanate monomers into polyfunctional isocyanate tri-condensate present in the composition is over 8%, preferably 10%, advantageously 15%,
- at least 1% and no more than 99% of biuret, preferably at least 2% and not more than 75%, are present, these quantities being given by weight.

**18.** A polyfunctional isocyanate tri-condensation product according to either of claims 16 to 17 **characterised in that** theallophanate comprises mono-, bis- and tris- allophanates in a quantity of advantageously 2/3, preferably at least 75% and even more preferably at least 90% by weight in relation to the total weight of the allophanate composition after removal of the unreacted monomers.

**19.** A polyfunctional isocyanate tri-condensation product according to any one of claims 16 to 18, **characterised in**

**that** the quantity of bis-allophanate represents up to 10%, even up to 20%, of the total weight of the allophanate composition.

**20.** A polyfunctional isocyanate tri-condensation product according to any one of claims 16 to 19, **characterised in that** the quantity of tris-allophanates is less than or equal to 30%, advantageously 20%, preferably 15% by weight in relation to the total weight of the total composition of the composition.

**21.** A polyfunctional isocyanate tri-condensation product according to any one of claims 16 to 20, **characterised in that** the ratio

$$\frac{\text{bis-allophanate groups + tris-allophanate groups}}{\text{mono-allophanate groups}}$$

is equal to or greater than 0.1 and may be up to 0.3 or even up to 0.5.

**22.** A polyfunctional isocyanate tri-condensation product according to claim 16 comprising hexamethylene diisocyanate biuret.

**23.** Composition according to any one of claims 16 to 22, **characterised in that** the isocyanate(s) used for the (cyclo) condensation reaction is(are) different from the isocyanate(s) used for the allophanation reaction.

**24.** Use of a composition according to any one of claims 16 to 23 to produce a coating.

**Patentansprüche**

**1.** Verfahren zur Herstellung einer Zusammensetzung von polyfunktionellen Isocyanat-Trikondensaten, die vorzugsweise mindestens eine Isocyanurat- und/oder Biuretgruppe umfasst, umfassend die Schritte, dass:

einem polyfunktionellen Isocyanat-Trikondensat oder einem Gemisch aus verschiedenen polyfunktionellen Isocyanat-Trikondensaten, das/die durch (Cyclo)kondensation, insbesondere durch (Cyclo)trimerisierung eines oder mehrerer identischer oder verschiedener monomerer Isocyanate und ggf. eines anderen Monomers erhalten wurde/n, ein Allophanat aus einem oder mehreren identischen oder verschiedenen Isocyanaten oder ein Gemisch aus verschiedenen Allophanaten zugesetzt wird, wobei die Isocyanate oder monomeren Isocyanatgemische, die zur Herstellung des/der polyfunktionellen Isocyanats/e verwendet werden, identisch mit dem/den zur Herstellung des/der Allophanats/Allophanate verwendeten Isocyanat/en oder Isocyanatgemisch sind oder davon verschieden sind, und das/die Allophanat/e ausgehend von unverzweigten Alkylalkoholen von $C_4$ bis $C_8$ hergestellt wird/werden.

**2.** Verfahren nach Anspruch 1,
**dadurch gekennzeichnet, dass**
die polyfunktionellen Isocyanat-Trikondensate der folgenden allgemeinen Formel entsprechen:

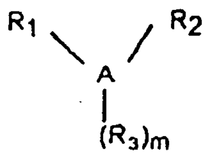

(I)

in der A das Folgende darstellt:

- eine Isocyanuratgruppe mit der Formel:

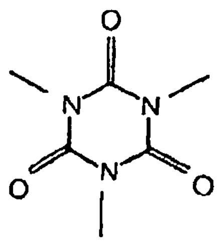

- eines ihrer Derivate, wie die Iminooxadiazindione mit der folgenden Formel:

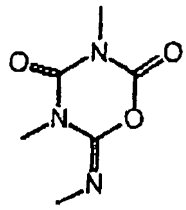

- eines ihrer Derivate, wie die Oxadiazintrione mit der folgenden Formel:

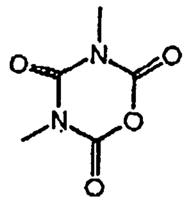

- eine Biuretgruppe mit der folgenden Formel:

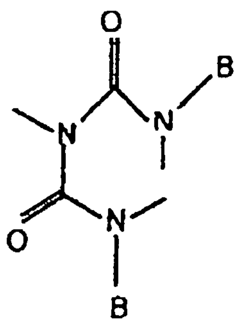

wobei B H oder eine Kohlenwasserstoffgruppe ist, das heißt Kohlenstoff und Wasserstoff sowie möglicherweise andere Atome (O, S, Si,...) enthält, die vorzugsweise zwischen 1 und 20 Kohlenstoffatome aufweist; oder

- eine Gruppe mit der folgenden Formel:

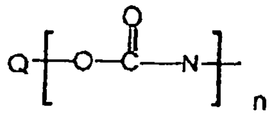

und in der identische oder verschiedene $R_1$, $R_2$, $R_3$ eine Kohlenwasserstoffgruppe, insbesondere eine aliphatische, cycloaliphatische, heterocyclische oder aromatische, darstellen, die eine echte oder abgeleitete Isocyanatfunktion umfasst, Q eine Kohlenwasserstoffgruppe, vorzugsweise Alkyl, ist, wie für $R_1$ bis $R_3$ definiert,
m eine ganze Zahl von 0 bis 2 ist,
n eine ganze Zahl von 3 bis 4 ist.

**3.** Verfahren nach einem der vorhergehenden Ansprüche, wobei die Zusammensetzung der polyfunktionellen Isocyanat-Trikondensate mindestens ein echtes polyfunktionelles Isocyanat-Isocyanurat umfasst.

**4.** Verfahren nach einem der vorhergehenden Ansprüche, wobei die Allophanate der folgenden allgemeinen Formel II entsprechen:

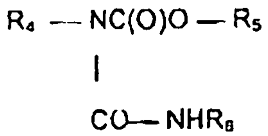

(II)

in der:

- verschiedene oder identische $R_4$ und $R_6$ eine Kohlenwasserstoffgruppe, insbesondere eine aliphatische, cycloaliphatische, heterocyclische oder aromatische, darstellen, die eine echte oder abgeleitete Isocyanatfunktion umfasst
- $R_5$ eine Alkylgruppe, das heißt der Rest einer unverzweigten $C_4$ bis $C_8$-Alkoholverbindung nach Entfernung der OH-Funktion ist.

**5.** Verfahren nach einem der vorhergehenden Ansprüche,
**dadurch gekennzeichnet, dass**
den polyfunktionellen Isocyanat-Trikondensaten ein Gemisch aus Allophanaten zugesetzt wird, das ein primäres Allophanat umfasst, das vorzugsweise etwa 1/4, bevorzugter etwa 1/3, noch bevorzugter etwa 2/5 (in Masse) primäres/primäre Allophanat/e umfasst.

**6.** Verfahren nach einem der vorhergehenden Ansprüche,
**dadurch gekennzeichnet, dass**
das Gemisch aus Allophanaten Mono-, Bis- und Tris-Allophanate umfasst, vorzugsweise in einer Menge von mindestens 2/3, bevorzugter von mindestens 75 Gew.-% und noch bevorzugter von mindestens 90 Gew.-% im Verhältnis zum Gesamtgewicht der Verbindung aus Allophanaten nach Entfernen der Monomere, die nicht reagiert haben.

**7.** Verfahren nach einem der vorhergehenden Ansprüche,
**dadurch gekennzeichnet, dass**
die Menge an Bis-Allophanat bis zu 10 %, sogar bis zu 20 % des Gesamtgewichts der Allophanatverbindung darstellt.

**8.** Verfahren nach einem der vorhergehenden Ansprüche,
**dadurch gekennzeichnet, dass**
die Menge an Tris-Allophanaten kleiner oder gleich 30 Gew.-%, vorzugsweise 20 Gew.-%, bevorzugter 15 Gew.-% im Verhältnis zum Gesamtgewicht der Gesamtzusammensetzung der Zusammensetzung beträgt.

**9.** Verfahren nach einem der vorhergehenden Ansprüche,
**dadurch gekennzeichnet, dass**

das Verhältnis

$$\frac{\text{Bis-Allophanatfunktionen + Tris-Allophanatfunktionen}}{\text{Mono-Allophanatfunktionen}}$$

gleich oder größer als 0,1 ist und bis zu 0,3, sogar bis zu 0,5 betragen kann.

**10.** Verfahren zur Herstellung einer Zusammensetzung aus polyfunktionellen Isocyanat-Trikondensaten mit geringer Viskosität nach Anspruch 1, welche Isocyanurat- und/oder Biuretgruppen umfasst, ausgehend von Isocyanaten, das die folgenden Schritte a) und b) in einer beliebigen Reihenfolge umfasst:

a) katalytische (Cyclo)kondensation eines oder mehrerer erster identischer oder verschiedener monomerer Isocyanate und ggf. eines oder mehrerer identischer oder verschiedener Monomere bis zum Erhalt der vorgesehenen Umsetzungsrate;
b) Reaktion eines oder mehrerer zweiter monomerer Isocyanate, die miteinander identisch oder voneinander verschieden sind und mit dem/den ersten monomeren Isocyanat/en identisch oder verschieden davon ist/ sind, mit einem unverzweigten $C_4$ bis $C_8$-Alkylalkohol zur Bildung eines Carbamats, wobei die Reaktion ggf. katalysiert wird, und gleichzeitige oder nachfolgende Reaktion des Carbamats mit einem oder mehreren dritten monomeren Isocyanat/en, die voneinander verschieden oder miteinander identisch sind und mit den vorhergehenden identisch oder davon verschieden sind, um ein Allophanat oder ein Gemisch aus Allophanaten zu erhalten;

und die Schritte c) und d) in einer beliebigen Reihenfolge:

c) Mischen des Reaktionsprodukts des Schritts a) mit dem gesamten Reaktionsprodukt von Schritt b) oder einem Teil davon; und ggf.
d) Entfernen der Monomere (Isocyanate).

**11.** Verfahren nach Anspruch 1 oder 2,
**dadurch gekennzeichnet, dass**
das/die für die (Cyclo)kondensationsreaktion verwendete/n Isocyanat/e identisch mit dem/ den für die Allophanatisierungsreaktion verwendeten Isocyanat/en ist/ sind.

**12.** Verfahren nach Anspruch 1 oder 2,
**dadurch gekennzeichnet, dass**
das/die für die (Cyclo)kondensationsreaktion verwendete/n Isocyanat/e von dem/den für die Allophanatisierungsreaktion verwendeten Isocyanat/ en verschieden ist/ sind.

**13.** Verfahren nach einem der vorhergehenden Ansprüche,
**dadurch gekennzeichnet, dass**
das/die für die Allophanatisierungsreaktion verwendete/n Isocyanat/e und das/die für die Cyclotrimerisierungsreaktion verwendete/n Isocyanat/e mindestens eine, vorzugsweise zwei, bevorzugter drei der folgenden Bedingungen erfüllt/ erfüllen:

- mindestens eine, vorzugsweise zwei der NCO-Funktionen sind über einen gesättigten Kohlenstoff ($sp^3$) mit einem Kohlenwasserstoffgerüst verbunden;
- mindestens einer, vorzugsweise zwei der gesättigten Kohlenstoffe ($sp^3$) ist/ sind Träger mindestens eines, vorzugsweise zweier Wasserstoffatoms/e;
- alle dazwischen liegenden Kohlenstoffe, über die die Isocyanatfunktionen mit dem Kohlenwasserstoffgerüst verbunden sind, sind gesättigte Kohlenstoffe ($sp^3$), die vorzugsweiser teilweise, bevorzugter vollständig Träger eines Wasserstoffatoms, noch bevorzugter zweier Wasserstoffatome sind.

**14.** Verfahren nach einem der Ansprüche 10 bis 13,
**dadurch gekennzeichnet, dass**
das NCO/OH-Verhältnis in Schritt b) größer als 4, vorzugsweise größer als 6 ist.

**15.** Verfahren nach einem der vorhergehenden Ansprüche,
**dadurch gekennzeichnet, dass**
mindestens ungefähr 25 Gew.-% des Produkts von Schritt b) mit dem Produkt von Schritt a) gemischt werden, welches HDT (ein Hexamethylen-Diisocyanat-Trimer) ist.

**16.** Zusammensetzung aus polyfunktionellen Isocyanat-Trikondensaten mit verringerter Viskosität, die mindestens ein echtes polyfunktionelles Isocyanat-Trikondensat und mindestens ein primäres Allophanat umfasst, das ausgehend von einem unverzweigten $C_4$ bis $C_8$-Alkylalkohol hergestellt wurde, wobei die Zusammensetzung
**dadurch gekennzeichnet ist, dass**
sie weniger als 10 %, vorzugsweise weniger als 8 %, bevorzugter weniger als 5 %, noch bevorzugter weniger als 4 % und, noch mehr zu bevorzugen, weniger als 3 %, und, am meisten zu bevorzugen, weniger als 2 % umfasst, wobei der Anteil bis zu weniger als 1 % von trimeren Allophanaten im Verhältnis zum Gesamtgewicht der Zusammensetzung umfassen kann.

**17.** Zusammensetzung nach Anspruch 16, die mindestens ein echtes polyfunktionelles Isocyanat-Trikondensat und mindestens ein primäres Allphohanat umfasst, das ausgehend von einem unverzweigten $C_4$ bis $C_8$-Alkylalkohol hergestellt wurde, wobei die Zusammensetzung mindestens eine, vorzugsweise mindestens zwei, bevorzugter drei und noch bevorzugter vier der folgenden Bedingungen erfüllt:

- ein Verhältnis G, das definiert ist durch:

$$G = \frac{\text{echte polyfunktionelle Isocyanat-Trikondensate aus der Kondensation von drei identischen oder verschiedenen, weder mit Carbamat noch mit Allophanat modifizierten Isocyanatmolekülen}}{\text{Summe der polyfunktionellen Isocyanatmoleküle, die Träger mindestens einer trimären Funktion sind, aus der Kondensation von drei identischen oder verschiedenen Isocyanatmolekülen}}$$

ist allgemein größer als 0,3, vorzugsweise größer als 0,4, noch bevorzugter größer als 0,5;
- ein Gewichtsverhältnis zwischen Allophanat/Allophanat + echtem Trimer ist zwischen 2,5 und 99% enthalten, vorzugsweise zwischen 3 und 60% und bevorzugter zwischen 3,5 und 40% enthalten,
- die Trimere stammen aus einer Trimerisierungsreaktion, für die die Umsetzungsrate des oder der identischen oder verschiedenen Isocyanatmonomer/e zu polyfunktionellen Isocyanat-Trikondensaten, die in der Zusammensetzung enthalten sind, größer als 8 %, vorzugsweise 10 %, bevorzugter 15 % ist,
- Biuret ist in einer Konzentration von mindestens 1 Gew.-% und höchstens 99 Gew.-%, vorzugsweise von mindestens 2 Gew.-% und höchstens 75 Gew.-% vorhanden.

**18.** Zusammensetzung aus polyfunktionellen Isocyanat-Trikondensaten nach einem der Ansprüche 16 und 17,
**dadurch gekennzeichnet, dass**
das Allophanat Mono-, Bis- und Tris-Allophanate in einer Menge von vorzugsweise mindestens 2/3, bevorzugter mindestens 75 Gew.-% und, noch bevorzugter, von mindestens 90 Gew.-% im Verhältnis zum Gesamtgewicht der Allophanatzusammensetzung nach dem Entfernen der Monomere, die nicht reagiert haben, umfasst.

**19.** Zusammensetzung aus polyfunktionellen Isocyanat-Trikondensaten nach einem der Ansprüche 16 bis 18,
**dadurch gekennzeichnet, dass**
die Menge an Bis-Allophanaten bis zu 10 %, sogar bis zu 20 % des Gesamtgewichts der Allophanatzusammensetzung darstellt.

**20.** Zusammensetzung aus polyfunktionellen Isocyanat-Trikondensaten nach einem der Ansprüche 16 bis 19,
**dadurch gekennzeichnet, dass**
die Menge an Tris-Allophanaten kleiner als oder gleich 30 Gew.-%, vorzugsweise 20 Gew.-%, bevorzugter 15 Gew.-% im Verhältnis zum Gesamtgewicht der Gesamtzusammensetzung der Zusammensetzung ist.

**21.** Zusammensetzung von polyfunktionellen Isocyanat-Trikondensaten nach einem der Ansprüche 16 bis 20, **dadurch gekennzeichnet, dass** das Verhältnis

$$\frac{\text{Bis-Allophanatfunktionen + Tris-Allophanatfunktionen}}{\text{Mono-Allophanatfunktionen}}$$

gleich oder größer als 0,1 ist und bis zu 0,3, sogar bis zu 0,5 betragen kann.

**22.** Zusammensetzung aus polyfunktionellen Isocyanat-Trikondensaten nach Anspruch 16, die Hexamethylen-Diisocyanat-Biuret umfasst.

**23.** Zusammensetzung nach einem der Ansprüche 16 bis 22, **dadurch gekennzeichnet, dass** das/die für die (Cyclo)kondensationsreaktion verwendete Isocyanat/e von dem/den für die Allophanatisierungsreaktion verwendeten Isocyanat/en verschieden ist/sind.

**24.** Verwendung einer Zusammensetzung nach einem der Ansprüche 16 bis 23 zur Ausführung einer Beschichtung.

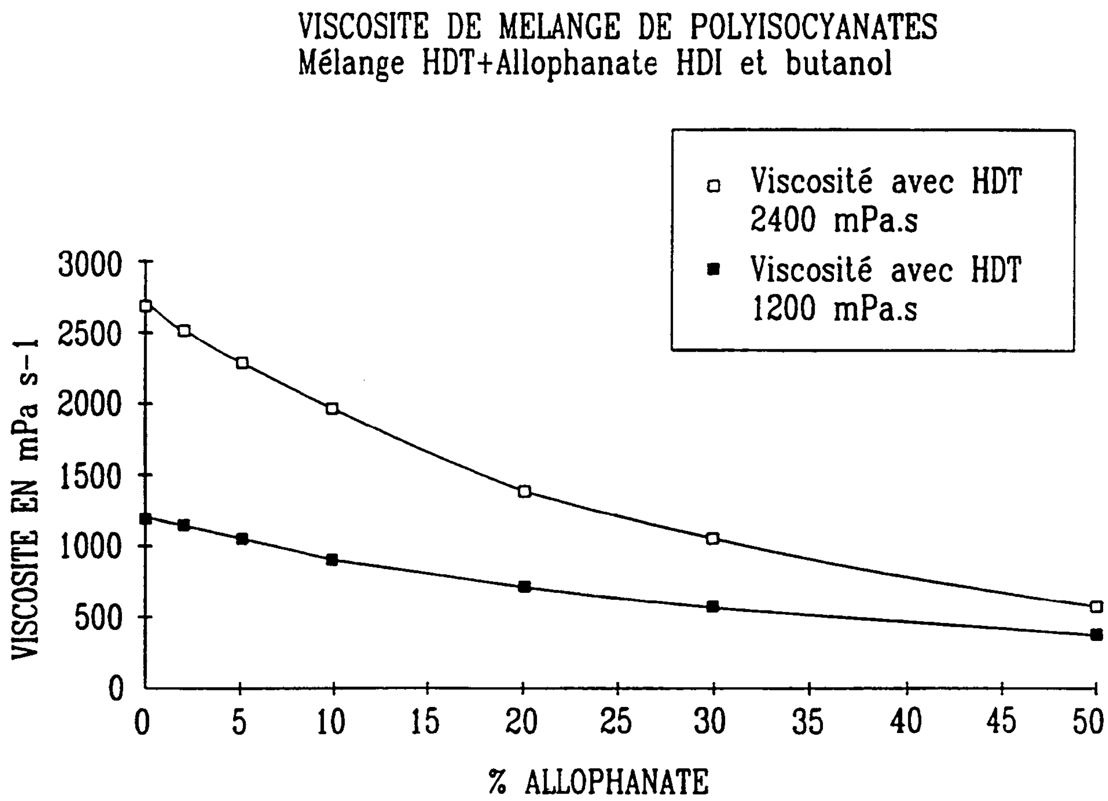
VISCOSITE DE MELANGE DE POLYISOCYANATES
Mélange HDT+Allophanate HDI et butanol
Viscosité avec HDT 2400 mPa.s
Viscosité avec HDT 1200 mPa.s
VISCOSITE EN mPa s-1
3000
2500
2000
1500
1000
500
0
0
5
10
15
20
25
30
35
40
45
50
% ALLOPHANATE

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 524500 A
- EP 524501 A
- EP 57653 A
- FR 8612524
- FR 2703357
- EP 691993 A